# EUROPEAN PATENT SPECIFICATION

(11) **EP 1 072 235 B1**
(45) Date of publication and mention of the grant of the patent: **10.12.2003**
(21) Application number: 00114831.1
(22) Date of filing: 11.07.2000
(51) Int. Cl.: A61F 2/34

(54) **Acetabular cup for hip prostheses**
Gelenkpfanne für Hüftprothesen
Coque acétabulaire pour prothèses de hanche

(30) Priority: 30.07.1999 IT UD990048 U
(43) Date of publication of application: 31.01.2001
(73) Proprietor: LIMA Lto SpA, 33030 Villanova di S. Daniele (UD) (IT)
(72) Inventor: Lualdi, Gabriele, 33034 Fagagna (UD) (IT)
(74) Representative: Petraz, Gilberto Luigi

(56) References cited:
- EP-A- 0 308 297
- WO-A-98/22049
- DE-U- 8 807 947
- DE-U- 29 614 342
- FR-A- 2 758 255
- GB-A- 2 159 416

## Description

### FIELD OF THE INVENTION

This invention concerns a acetabulum cup for hip prostheses as set forth in the main claim.

The acetabulum cup according to the invention is used in prostheses employed to restore femoral articulation in degenerative or traumatic pathologies of the hip joint.

### BACKGROUND OF THE INVENTION

The state of the art includes orthopaedic prostheses of the coxo-femoral articulation comprising an acetabulum cup, or artificial cotyloid cavity, which is inserted inside the natural acetabulum of the hip.

The artificial cotyloid cavity has a bell-shaped conformation, that is, substantially hemispherical, defining a containing seating on the inner side for a mating insert.

The insert, in turn, defines on the inner side a containing seating, substantially hemispherical in shape, inside which the spherical upper end, or head, of a femoral element is able to be housed, the femoral element being anchored to the femur in a conventional manner.

At present, the housing seating for the mating insert is made on the acetabulum cup in such a manner that the median longitudinal axis of the seating and that of the acetabulum cup coincide.

The rotary movements of the spherical head of the femoral element inside the relative containing seating are limited to defined angular values and consequently, the acetabulum cup must be applied perfectly into the acetabulum of the hip.

To be more exact, the acetabulum cup must be applied into the acetabulum of the hip in such a manner that its median longitudinal axis, and therefore the median longitudinal axis of the seating which it defines, is arranged inclined by a defined angle with respect to the front plane of the pelvis to allow the femur to move substantially as it does in the natural joint.

Often, however, the patient's bone morphology, also because of degeneration or the trauma suffered, does not allow the surgeon to position the acetabulum cup correctly and therefore the median longitudinal axis is positioned at an insufficient angle with respect to the front plane of the pelvis, thus limiting the movements of the femoral element.

An incorrect positioning of the median longitudinal axis, moreover, can cause the problem of an anomalous contact between the spherical head of the femoral element and the relative housing seating, with consequent problems of excess pressure, which can wear the surface of the seating and/or the spherical head can accidentally slip out, with serious problems for the patient.

The present Applicant has devised and embodied this invention to overcome these problems and to obtain other advantages.

### SUMMARY OF THE INVENTION

The invention is set forth and characterized in the main claim, while the dependent claims describe other characteristics of the invention.

The purpose of the invention is to achieve an acetabulum cup which will guarantee, irrespective of the patient's bone morphology or the degeneration or trauma which has occurred, a correct positioning of the housing seating of the insert with which the spherical head of the femoral element is coupled.

In accordance with this purpose, an acetabulum cup according to the invention is characterized in that it has a housing seating for said insert which, when at least a reference plane has been taken, has its median longitudinal axis inclined by a defined angle with respect to the median longitudinal axis of the acetabulum cup itself.

With the acetabulum cup according to the invention, therefore, even if a part of the hip is destroyed and the acetabulum cup has to be assembled with its median longitudinal axis too much inclined at the front with respect to the front plane of the pelvis, this is not a problem since the differences between the actual assembly angle and the theoretical assembly angle are compensated by the inclination of the housing seating by a value correlated to the difference between said angles.

The acetabulum cup according to the invention can be made in any material and have any outer conformation able to be attached to the bone seating.

According to the invention, the outer conformation comprises protruding parts such as fins or similar, able to be attached on the perimeter of the bone seating advantageously in correspondence with the damaged parts thereof.

The seating defined by the acetabulum cup can also have any conformation according to the conformation and the type of insert which it has to house.

### BRIEF DESCRIPTION OF THE DRAWINGS

These and other characteristics of the invention will be clear from the following description of a preferred form of embodiment, given as a non-restrictive example with reference to the attached Figures wherein:
- Fig. 1: is a view from above of an acetabulum cup according to the invention;
- Fig. 2: is a view from below of Fig. 1;
- Fig. 3: is a side view of Fig. 1;
- Fig. 4: is a longitudinal section of Fig. 3.

### DETAILED DESCRIPTION OF PREFERRED EMBODIMENT

The attached Figures show an acetabulum cup 10 according to the invention for hip prostheses.

The acetabulum cup 10 can be made of any material, for example titanium, and has a hemispherical outer conformation able to be inserted and attached in the acetabulum seating of the hip.

The attachment occurs in a conventional manner by means of screws, not shown here, which are inserted into through holes 11 and screwed into the bone tissue and/or by interference with the bone seating.

In this case, the acetabulum cup 10 according to the invention is equipped on a defined segment of the circumference with a protruding part 12 able to be coupled with any damaged part of the bone seating.

The acetabulum cup 10 according to the invention defines inside a seating 13 able to house a mating insert, not shown, which in turn defines inside a containing seating for the spherical head of a femoral element, which is also not shown in the drawings.

According to the main characteristic of the invention, the seating 13 has its median longitudinal axis 14 inclined by a defined angle α with respect to the median longitudinal axis 15 of the acetabulum cup 10.

In this way, even if the acetabulum cup 10 is installed with its median longitudinal axis 15 inclined with an angle which is different from the theoretical angle of assembly, due, for example, to a particular bone morphology of the seating, this is not a problem since the differences between the actual assembly angle and the theoretical assembly angle are compensated by said angle α, the value of which is precisely the difference between said actual and theoretical angles.

According to the invention, the angle α is between 5° and 30°, preferentially between 15° and 22°, advantageously around 18°.

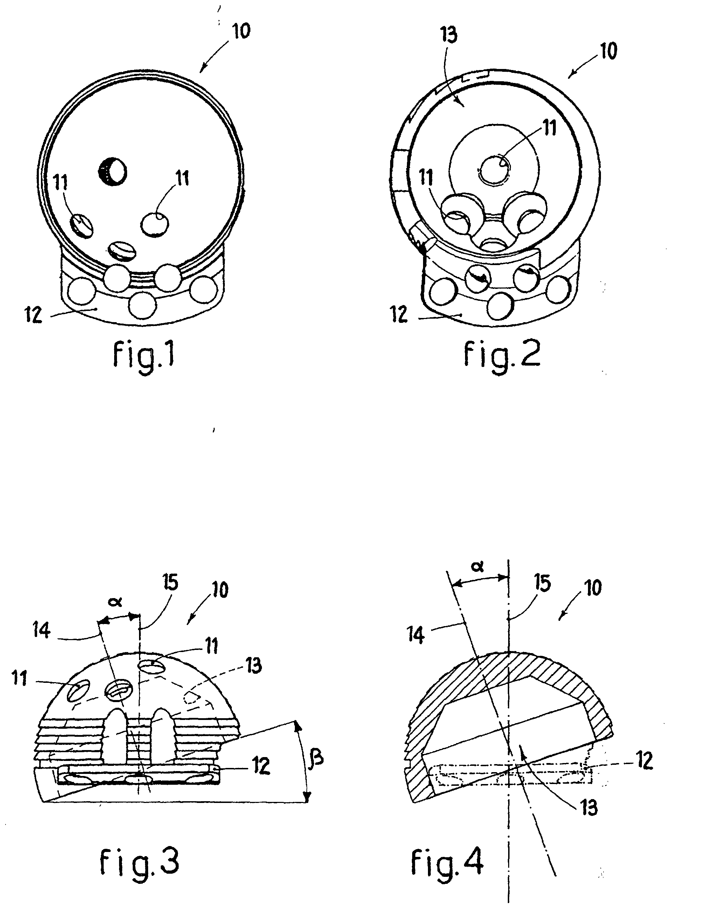

In this case, the acetabulum cup 10 shown here has a lower surface inclined by an angle β with respect to the horizontal (Fig. 3).

This angle β, which in the embodiment shown here is the same as the angle α, allows the femoral element to behave in a manner equivalent to what happens in a natural joint.

It is obvious that modifications and additions may be made to the acetabulum cup 10 as described heretofore.

For example, instead of being hemispherical its outer conformation can be cylindrical, conical, truncated cone or otherwise.

It is also obvious that, although the description refers to a specific example, a person of skill shall certainly be able to achieve many other equivalent forms of acetabulum cup, which fall within the scope of this invention as defined in the claims.

## Claims

1. Acetabulum cup for hip prostheses, said acetabulum cup being able to be inserted and attached in a bone cavity of the hip and defining inside a containing seating (13) for a mating insert able to couple with the upper end, or head, of a femoral element, the acetabulum cup comprising a protruding fin (12) for attaching the cup on the perimeter of said bone cavity in correspondence of a damaged part thereof so as to define a univocal mounting position of said acetabulum cup, **characterized in that** the median longitudinal axis (14) of said containing seating (13) is inclined by an angle (α) with respect to the median longitudinal axis (15) of the acetabulum cup (10) itself so as to define an inner part of said cup which is oriented forward with respect to said mounting position defined by said protruding fin (12).

2. Acetabulum cup as in Claim 1, **characterized in that** said angle (α) has a value which is the difference between the actual assembly angle and the theoretical assembly angle of the acetabulum cup (10) in said bone cavity of the hip.

3. Acetabulum cup as in Claim 1, **characterized in that** said angle (α) is between 5° and 30°.

4. Acetabulum cup as in Claim 3, **characterized in that** said angle (α) is equal to 18°.

5. Acetabulum cup as in Claim 1, **characterized in that** it has the lower surface inclined by an angle of (β) with respect to the horizontal.

6. Acetabulum cup as in Claim 5, **characterized in that** said angle (β) is equal to said angle (α).

## Patentansprüche

1. Gelenkpfanne für Hüftprothesen, wobei die Gelenkpfanne in einen Knochenhohlraum der Hüfte eingesetzt und in ihm angebracht werden kann und im Innern einen Aufnahmesitz (13) für einen passenden Einsatz definiert, der mit dem oberen Ende oder Kopf eines Oberschenkelknochenelements koppeln kann, wobei die Gelenkpfanne einen vorstehenden Steg (12) zur Anbringung der Pfanne auf dem Perimeter des Knochenhohlraums in Entsprechung eines beschädigten Teils desselben umfasst, um eine eindeutige Montageposition der Gelenkpfanne zu definieren, **dadurch gekennzeichnet, dass** die Längsmittelachse (14) des Aufnahmesitzes (13) um einen Winkel (α) in Bezug zur Längsmittelachse (15) der Gelenkpfanne (10) selbst geneigt ist, um einen inneren Teil der Pfanne zu definieren, der in Bezug zu der Montageposition, die durch den vorstehenden Steg (12) definiert ist, nach vorne orientiert ist.

2. Gelenkpfanne nach Anspruch 1, **dadurch gekennzeichnet, dass** der Winkel (α) einen Wert aufweist, der der Unterschied zwischen dem tatsächlichen Montagewinkel und dem theoretischen Montagewinkel der Gelenkpfanne (10) in dem Knochenhohlraum der Hüfte ist.

3. Gelenkpfanne nach Anspruch 1, **dadurch gekennzeichnet, dass** der Winkel (α) zwischen 5° und 30° ist.

4. Gelenkpfanne nach Anspruch 3, **dadurch gekennzeichnet, dass** der Winkel (α) gleich 18° ist.

5. Gelenkpfanne nach Anspruch 1, **dadurch gekennzeichnet, dass** sie die untere Oberfläche um einen Winkel von (β) in Bezug zur Horizontalen geneigt aufweist.

6. Gelenkpfanne nach Anspruch 5, **dadurch gekennzeichnet, dass** der Winkel (β) gleich dem Winkel (α) ist.

## Revendications

1. Cupule d'acétabulum pour des prothèses de hanches, ladite cupule d'acétabulum étant capable d'être insérée et attachée dans une cavité osseuse de la hanche et définissant à l'intérieur une embase contenante (13) pour un insert d'accouplement capable de se coupler avec l'extrémité supérieure, ou la tête, d'un élément fémoral, la cupule d'acétabulum comprenant une ailette protubérante (12) destinée à attacher la cupule sur le périmètre de ladite cavité osseuse en correspondance avec une partie endommagée de celle-ci de façon à définir une position de montage univoque de ladite cupule d'acétabulum, **caractérisée en ce que** l'axe longitudinal médian (14) de ladite embase contenante (13) est inclinée d'un angle (α) par rapport à l'axe longitudinal médian (15) de la cupule d'acétabulum (10) elle-même de façon à définir une partie interne de ladite cupule qui est orientée vers l'avant par rapport à ladite position de montage définie par ladite ailette protubérante (12).

2. Cupule d'acétabulum selon la revendication 1 **caractérisée en ce que** ledit angle (α) a une valeur qui est la différence entre l'angle d'assemblage effectif et l'angle d'assemblage théorique de la cupule d'acétabulum (10) dans ladite cavité osseuse de la hanche.

3. Cupule d'acétabulum selon la revendication 1, **caractérisée en ce que** ledit angle (α) est compris entre 5° et 30°.

4. Cupule d'acétabulum selon la revendication 3, **caractérisée en ce que** ledit angle (α) est égal à 18°.

5. Cupule d'acétabulum selon la revendication 1, **caractérisée en ce que** sa surface inférieure est inclinée d'un angle (β) par rapport à l'horizontale.

6. Cupule d'acétabulum selon la revendication 5, **caractérisée en ce que** ledit angle (β) est égal audit angle (α).
